# EUROPEAN PATENT APPLICATION

(11) **EP 0 593 251 A1**
(43) Date of publication of application: **20.04.1994**
(21) Application number: 93308103.6
(22) Date of filing: 12.10.1993
(51) Int. Cl.: C07C 231/12, C07C 209/74, C07C 211/52

(54) **Process for the catalytic debromination of halogenated monocyclic aromatic compounds**

(30) Priority: 13.10.1992 US 959848
(71) Applicant: HOECHST CELANESE CORPORATION, Somerville, N.J. 08876 (US)
(72) Inventor: Lee, William Robert, Cranston, RI 02910 (US); Sadanani, Narayan D., West Warwick, RI 02893 (US); Kelly, Michael G., Coventry, RI 02816 (US)
(74) Representative: De Minvielle-Devaux, Ian Benedict Peter

(57) **Abstract**

A process for the bromination of a monocyclic aromatic compound and its regiospecific chlorination. The halogenation reactions are carried out in a protonic solvent which is replaced by a polar solvent without isolation of the halogenation reaction product. The halogenation reaction product is then catalytically debrominated.

## Description

### BACKGROUND OF THE INVENTION

### Technical Field

This invention is directed to the regiospecific chlorination of a substituted monocyclic aromatic compound.

### Background Art

Bromine and chlorine are used in organic synthesis as protective groups to block ring positions in benzene derivatives in order to obtain regiospecific substitution on the benzene ring. Bromine is also used as a protective group to obtain regiospecific chlorination of an aromatic ring. In either case the protective group or groups may be removed by catalytic hydrogenolysis of the carbon-halogen bond. Examples of the use of bromine and chlorine as protective groups are found in the literature; e.g. Ghosh, R. and R. Robinson, J. Chem. Soc., 506 (1944) and Blatt, A.H., N. Gross, and E.W. Tristam, J. Org. Chem., 22, 1588 (1957). These articles also describe the catalytic hydrogenolysis of the carbon-halogen bond; see also House, H. Modern Synthetic Reactions 1-22 (1967). The teachings of these references are hereby incorporated by reference.

Bromination and chlorination reactions are best conducted in a protonic solvent such as acetic acid. However, the catalytic hydrogenolysis (dehalogenation) reaction does not occur very well in an acetic acid reaction medium. It has also been established that a polar solvent such as methanol is required for the catalytic dehalogenation step to efficiently proceed. In the past, prior to the catalytic dehalogenation step, the halogenated precursor was isolated by drowning the reaction mixture into water and recovering the resulting precipitate. This isolation step led to significant environmental problems because of the large amount of waste acidic water requiring waste water treatment. In addition, fresh acetic acid was required for each batch adding significantly to the cost of raw materials. The presence of water in the intermediate (halogenated precursor) also has an adverse impact on the solubility of the precursor and the selectivity of the dehalogenation reaction. Therefore, the precursor had to be dried prior to the catalytic (hydrogenolysis) removal of the halogen from the product, which led to significant odor problems. It is apparent that the isolation and drying of the intermediate product prior to the catalytic dehalogenation step increased costs and reduced productivity.

This invention eliminates the above cited problems and it provides a more efficient, high productivity, lower cost and more environmentally sound process for introducing and removing halogen protective groups in a multi-step chemical synthesis.

### SUMMARY OF THE INVENTION

This invention comprises a process for brominating substituted monocyclic aromatic compound in a protonic solvent such as an organic acid for the purpose of blocking substituent positions on the aromatic ring and then regiospecifically introducing chlorine into the ring at the unblocked positions. The bromine protective group is then removed from the ring by catalytic hydrogenolysis; preferably in the presence of an alkaline reagent.

In the process of the invention, prior to the debromination step, the protonic reaction solvent is exchanged with a polar solvent without isolating the halogenated intermediate. After the solvent exchange is accomplished, the bromine protective group may be removed from the aromatic ring by catalytic dehydrogenolysis of carbon - bromine bond.

The solvent exchange step of the process comprises heating the reaction mixture to remove the protonic reaction solvent and a polar solvent is added to form a solution of the halogenated reaction product. Optionally, the reaction mixture may be cooled prior to the addition of the polar solvent.

The protonic reaction solvent (preferably acetic acid) may be removed by evaporation and the reaction solvent is exchanged at an elevated temperature to dissolve the precursor in a polar solvent (e.g. methanol). The ability to exchange the solvent was surprising and unexpected because the aromatic halogenated precursors generally have a melting point of greater than 150°C - 170°C. It was, expected that the precursor would crystallize out of solution during evaporation of protonic solvent which would have prevented substantial removal of the protonic solvent. However, the precursor surprisingly remains molten during the distillation even at low solvent concentration and it is possible to remove up to about 95% of the protonic solvent. After reducing the protonic solvent concentration, a polar solvent, (e.g. methanol) is then added and the molten reaction product goes smoothly into solution. Addition of the polar solvent also serves to cool the reaction mixture, prior to the catalytic debromination (hydrogenation) step. The ability to conduct this distillation and solvent exchange was completely unexpected based on the physical properties of the halogenated intermediate, since it was expected that the reaction mixture would solidify at low solvent concentration.

### DESCRIPTION OF THE PREFERRED EMBODIMENTS

The invention is a process for preparing regiospecifically chlorinated benzene derivatives. The invention comprises the steps of introducing one or more bromine protective groups into a substituted monocyclic aromatic compound in a protonic solvent as the reaction medium; chlorinating the bromine substituted benzene derivative; exchanging the protonic solvent with a polar solvent without isolation of the halogenated reaction product and catalytically hydrogenating the halogenated reaction product to cleave the carbon-bromine bond.

The bromine protective group will react at the most reactive ring positions on the monocyclic aromatic compound. It will be readily apparent to one skilled in the art that by adjusting the molar ratio of bromine to aromatic compound and the reaction conditions, one can control the number of ring positions brominated. After bromination, the chlorination step may be conducted using the same general principles. Similarly, when the starting monocyclic aromatic compound contains a halo-reactive substituent; the halo-reactive substituent may be protected; e.g. an amine substituent would be acylated prior to the halogenation reactions. Optionally, it may be deacylated after the debromination step or recovered in its acylated form.

The monocyclic aromatic compounds useful in the process of this invention may be represented by the general formula: 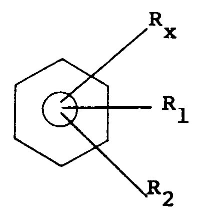
wherein: R represents C₁ to C₄ alkyl, C₁ to C₄ alkoxy, chlorine, fluorine, -NHCOCH₃, -CONHCOCH₃; -HNCO(CH₂)_{y} CH₃ wherein x is selected from an integer of 1 to 3; and y is 0 to 3; and
R₁ and R₂ are independently selected from H, C₁ to C₄ alkyl, C₁ to C₄ alkoxy, chlorine and fluorine;

Exemplary starting materials within the above formula and their chloro derivatives according to the process of this invention are set forth in Table 1. The chlorination products are listed in their free amine form.

**Table 1**

| Starting Material | Chlorination Product |
|---|---|
| Aniline | 2,6-Dichloroaniline |
| Acet-m-toluidine [N-(3-methylphenyl]acetamide] | 2,6-Dichloro-m-toluidine |
| [N-(3-methylphenyl)propionamide] | 2,6-Dichloro-m-toluidine |
| Phenol | 2,6-Dichlorophenol |
| 3-Methylphenol (m-Cresol) | 2,6-Dichloro-3-methyl phenol |
| [N-(3-ethylphenyl)acetamide] | 2,6-Dichloro-3-ethylaniline |
| [N-(3-ethylphenyl)propionamide] | 2,6-Dichloro-3-ethylaniline |
| 3-Ethylphenol | 2,6-Dichloro-3-ethylphenol |
| 2-Chloroaniline | 2,6-Dichloroaniline |
| Acet-o-toluidine [N-(2-methylphenyl)acetamide] | 2-Chloro-6-methylaniline |
| [N-(2-ethylphenyl)acetamide] | 2-Chloro-6-ethylaniline |
| 2-Ethylphenol | 2,-Chloro-6-ethylphenol |
| 2-Fluoroaniline | 2-Chloro-6-Fluoroaniline |
| 2-Methylphenol (o-cresol) | 2-Chloro-6-methylphenol |
| Anisole (Methylphenylether) | 2,6-Dichloro Anisole (2,6-Dichlorophenyl methylether |
| 3-Methylanisole (3-Methylphenyl methylether) | 2,6-Dichloro-3-methyl anisole |

The protonic solvent useful in the process of the invention may be selected from an aliphatic C₁ to C₅ carboxylic acids and mixtures thereof; preferably acetic acid or propionic acid; most preferably acetic acid. The polar solvent useful in the invention is selected from C₁ to C₅ alcohols and mixture thereof preferably methanol, ethanol and isopropanol. The reaction solvents can be used in combination with an inert solvent such as hexane, octane, a halogenated hydrocarbon such as trichloromethane, tetrachlorethane etc.

The following general procedure illustrates the invention using 1.0 mole of monocyclic aromatic compound as the basis. About 1.0 mole of monocyclic aromatic compound is dissolved in acetic acid and 0.5 moles bromine is added. This addition is then followed by addition of 0.6 moles of 50% hydrogen peroxide which oxidizes any HBr formed to Br₂.

After stirring 30 minutes, 1.352 moles of chlorine gas are introduced subsurface over 2-3 hours while maintaining the reaction temperature between 35 to 65°C, preferably between 35 to 45°C. The chlorination is completed by another addition of 1.06 moles of 50% hydrogen peroxide to generate chlorine in situ from the HCI in the reaction mixture. The reaction mixture is held for 30 additional minutes, and then the excess peroxide is destroyed by adding sodium bisulfite until a negative peroxide test is achieved.

After destroying the peroxide, the reaction flask is fitted for distillation with a Claisen head and condenser and the acetic acid is distilled off until the pot temperature reaches 130° to 150°, preferably 140°C. The concentrated reaction mixture is cooled to approximately to 100° to 120°C and methanol is added at a controlled rate so that the reflux does not flood the condenser. The warm solution is clarified to remove inorganic salts and transferred to an autoclave for debromination. Slightly less than one equivalent of sodium hydroxide is added for neutralization of the liberated hydrogen bromide, and 3 to 5% palladium on carbon catalyst is added. Debromination is carried out using hydrogen. The product is isolated by stripping off as much of the methanol as possible under atmospheric pressure, and adding water to precipitate the product. The debromination material is isolated by filtering on a Buchner funnel. The filter cake may be used in subsequent processing step if the wet cake is suitable or it may be dried.

### EXAMPLE 1

This example illustrates the preparation of 2,6-dichloro-3-methylacetanilide. (Formula: C₉H₉Cl₂NO, MW: 218.1). Acetic anhydride is added with stirring to a solution of m-Toluidine in acetic acid. The temperature rises to 60°C then falls. After 30 minutes the solution is diluted with acetic acid and bromine is added over 30 minutes at 35-45°C followed by 50% Hydrogen peroxide over 30 minutes at 45-60°C. After stirring 30 minutes chlorine is added subsurface over 2-3 hours followed by 50% hydrogen peroxide over 60 minutes at 45-60°C. After 30 minutes at 55-60°C, the acetic acid is distilled off, until the pot temperature reaches 140°C. Methanol is added at 110°C to give a solution. The warm solution is transferred to a Parr apparatus and debrominated under 50-500 psig hydrogen pressure, using 3% palladium on carbon catalyst (load 1-5% based on starting material). The catalyst is removed by filtration, most of the methanol is distilled off and the product is precipitated by addition of water and finally filtered on a buchner funnel. The yield is 74.4%.

### EXAMPLE 2

This example illustrates the preparation of 2,4-dichlorotoluidine (2,6-dichloro-3-methylaniline, Formula: C₇H₇Cl₂N MW: 176.1) from the product of Example 1. 2,6-dichloro-3-methylacetanilide is added over 0.5 hours to 65-70% sulfuric acid. The reaction is held at 105°C for 4 hours. The solution is cooled to 80°C and diluted with an equal volume of water. The product is steam distilled and isolated by phase separation. The crude reaction product is vacuum distilled at 10mm through a 28 cm packed column to give 2,4 dichlorotoluidine having a purity of 99+% and the reaction yield is 87.4%. The overall product yield of Example 1 and 2 was 65%.

The foregoing detailed description is given to illustrate the invention and no unnecessary limitations should be implied. Various modifications of the invention will be obvious to those skilled in the art.

## Claims

1. A process for regiospecifically chlorinating a monocyclic aromatic compound of the formula: 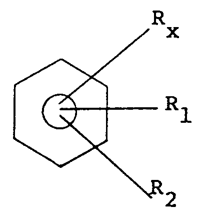 wherein: R represents C₁ to C₄ alkyl, C₁ to C₄ alkoxy, chlorine, fluorine, -CONHCOCH₃; CH₃(CH₂)_{y}CONH- wherein x is selected from an integer of 1 to 3; and y is 0 to 3; and R₁ and R₂ are independently selected from H, C₁to C₄ alkyl, C₁ to C₄ alkoxy, chlorine and fluorine; which comprises dissolving said compound in a protonic solvent; brominating the most active ring positions of said compound to block one or more predetermined ring position; chlorinating one or more of the remaining unblocked ring positions; exchanging the protonic solvent with a polar solvent without isolating said halogenated monocyclic compound and debrominating said compound by catalytic hydrogenolysis.

2. A process according to claim 1 wherein said monocyclic aromatic compound is 3-methylacetanilide.

3. A process according to claim 1 or 2 wherein said protonic solvent is selected from C₁ to C₅ carboxylic acids and mixtures thereof.

4. A process according to claim 3 wherein said carboxylic acids are acetic acid and/or propionic acid.

5. A process according to any of claims 1-4 wherein said polar solvent is selected from C₁ to C₅ alcohols and mixtures thereof.

6. A process according to claim 5 wherein said alcohols are methanol and/or ethanol and/or propanol and/or isopropanol.
